(19) Europäisches Patentamt
European Patent Office
Office européen des brevets

(11) Publication number: **0 089 231**
**B1**

(12) **EUROPEAN PATENT SPECIFICATION**

(45) Date of publication of patent specification: **20.05.87**

(51) Int. Cl.$^4$: **G 03 C 1/06**, G 03 C 1/10, G 03 C 1/28, G 03 C 1/30, G 03 C 5/16, C 07 D 513/02

(21) Application number: **83301427.7**

(22) Date of filing: **15.03.83**

(54) **Silver halide photographic material.**

(30) Priority: **15.03.82 JP 40382/82**

(43) Date of publication of application:
**21.09.83 Bulletin 83/38**

(45) Publication of the grant of the patent:
**20.05.87 Bulletin 87/21**

(84) Designated Contracting States:
**DE FR GB**

(56) References cited:
**DE-A-2 133 053**
**DE-A-2 151 095**
**DE-A-2 422 772**
**GB-A- 805 826**
**US-A-2 240 472**
**US-A-3 026 202**
**US-A-3 773 731**

(73) Proprietor: **KONISHIROKU PHOTO INDUSTRY CO. LTD.**
**No. 26-2, Nishishinjuku 1-chome Shinjuku-ku**
**Tokyo 160 (JP)**

(72) Inventor: **Machida, Katsutoshi**
**972-4 Yokokawa-cho**
**Hachioji-shi Tokyo 193 (JP)**
Inventor: **Sakuma, Haruhiko**
**65-4 Takakura-cho**
**Hachioji-shi Tokyo 192 (JP)**
Inventor: **Ishikawa, Naooki**
**7-17 Takagi-cho**
**Kokubunji-shi Tokyo 185 (JP)**
Inventor: **Kurihara, Seiji**
**2-5 Ohwada-cho**
**Hachioji-shi Tokyo 192 (JP)**

(74) Representative: **Ellis-Jones, Patrick George Armine et al**
**J.A. KEMP & CO. 14 South Square Gray's Inn**
**London WC1R 5EU (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 089 231 B1**

**Description**

The present invention relates to a silver halide photographic material that is designed to produce an image having an minimum quality deterioration and providing stable photographic characteristics.

Recent versions of silver halide photographic materials are required to satisfy various needs for high performance, especially stable photographic characteristics for high to superhigh sensitivity materials. In particular, X-ray films that involve human exposure to X-rays are required to have a sensitivity high enough to produce more information with less exposure to X-rays without increasing fog and sacrificing good image quality.

One conventional method for increasing the sensitivity of silver halide photographic materials is described in "The Theory of the Photographic Process", 3rd ed., Macmillan, 1967, pp. 369—370 and consists of incorporating polyalkylene oxide, especially polyethylene oxide compounds, in silver halide photographic materials. Many researchers have reported that polyalkylene oxide compounds are also useful as antistats to prevent troubles due to static buildup in the photographic materials (e.g. static marks and dust collection). Also reported in many papers is the use of polyalkylene oxide surfactants to enable uniform coating of layers in the photographic material such as photosensitive layers and protective layers.

In spite of the many advantages they provide for silver halide photographic materials, polyalkylene oxide compounds have one great problem in that if their concentration exceeds a certain limit, the resulting image has reduced quality and its photographic characteristics are greatly impaired. This problem is particularly serious with X-ray films which must be rapidly processed (within 20 to 50 seconds) at high pHs under elevated temperatures (30 to 40°C). Many studies have been made on the method of producing silver halide photographic materials that withstand the severe conditions employed for rapid processing at elevated temperatures. One typical method is to use a "hardener" for hardening gelatin used as a binder in the photographic material. Known hardeners are inorganic compounds such as chrom alum, as well as organic compounds such as aldehyde compounds (e.g. formaldehyde and glutaraldehyde), compounds having active halogens as described in U.S. Patent No. 3,288,755, compounds having reactive ethylenically unsaturated bonds as described in U.S. Patent No. 3,635,718, epoxy compounds of the type described in U.S. Patent No. 3,091,537, and halogen carboxyaldehydes such as mucochloric acid. But if these hardeners are incorporated in the silver halide photographic material in the amount necessary for making the material withstand rapid processing at increased temperatures, they cause adverse effects on the properties of the photographic material by increasing the fog, decreasing the sensitivity, varying the gradation or reducing the maximum density, or their hardening power changes with time to cause "after-hardening". Another method for preparing a silver halide photographic material capable of producing a quality image after rapid processing under elevated temperatures is to incorporate more gelatin (binder) with respect to the amount of silver in the photographic material, but this method either reduces the sensitivity or renders the photographic material less compatible with development, fixing or washing procedures. Therefore, it has long been desired to devise a technique for preparing a silver halide photographic material that allows the use of a sufficient amount of a polyalkylene oxide compound for producing a quality image by rapid processing at elevated temperatures.

The object of the present invention is to provide a silver halide photographic material capable of minimizing the deterioration of the quality of a photographic image even when a polyalkylene oxide compound which imparts very effective characteristics to the silver halide photographic material is used in sufficient amount enabling the material to be capable of producing a quality image when it is subjected to rapid processing at high pHs and under elevated temperatures.

These objects of the present invention can be accomplished by a silver halide photographic material comprising a support and one or more layers thereon, at least one layer containing (a) at least one compound of formula (I) and (b) at least one compound having an alkylene oxide chain with an average molecular weight of 250 or more which is a polyalkylene glycol or a condensation product of alkylene oxide and an organic compound having active hydrogen, the compound of formula (I) being present in an amount from 0.001 to 2 mg per gram of total binder present in a hydrophilic colloidal layer.

Formula (I)

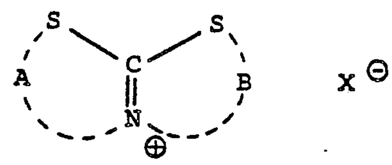

I

wherein A and B each independently represent the nonmetallic atoms necessary for forming a hetero ring; and X is a negative ion (e.g. $Cl^-$, $Br^-$, $ClO_4^-$ or $CH_3SO_3^-$).

The present invention is characterized by incorporating at least one polyalkylene oxide compound and a compound of formula (I) in combination in one or more of the constituent layers of a silver halide photographic material. At least one compound of each type may be incorporated in at least one constituent

layer, or the two compounds may be incorporated separately in two or more constituent layers on the same side of the support on which the silver halide emulsion layers are formed. The objects of the present invention can be achieved in either way. In a preferred embodiment, the compound of formula (I) (hereunder referred to as compound A) is added to a silver halide emulsion layer and the polyalkylene oxide compound is incorporated in the same emulsion layer and/or a non-sensitive layer.

Preferred compounds (A) are such that groups A and/or B or non-metallic atoms necessary to form a hetero ring are represented by

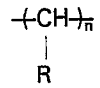

wherein R is a hydrogen atom or a lower alkyl group, generally of 1 to 6 carbon atoms; and n is 2 or 3.

Compounds (A) to be used in the present invention are most preferred in the case where non-metallic atoms A necessary to form a hetero ring have the following formula:

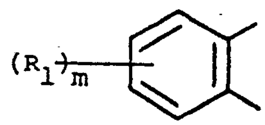

wherein $R_1$ is a hydroxyl group, a halogen atom, an alkyl group which may have a substituent, an aralkyl group which may have a substituent, an acyl group which may have a substituent, a carboxymethyl group which may have a substituent, or a —COOH or —$SO_3H$ group; and m is 0, 1 or 2, and where non-metallic atoms B are as defined above.

Typical examples of compounds (A) that can be used in the present invention are listed below.

[A-1] 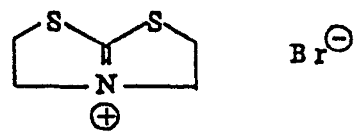

[A-2] 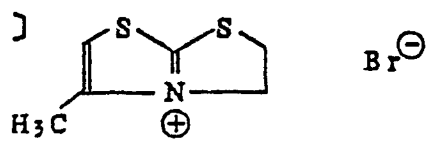

[A-3] 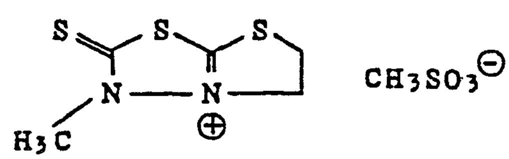

[A-4] 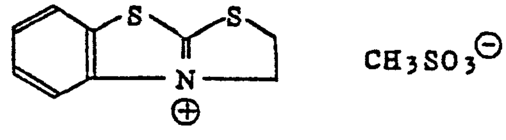

[A-5] 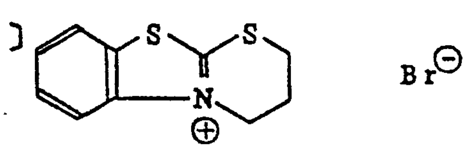

[A—6] 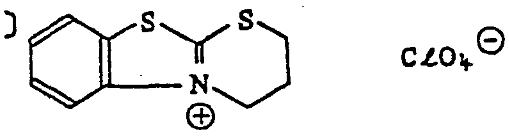

[A—7] 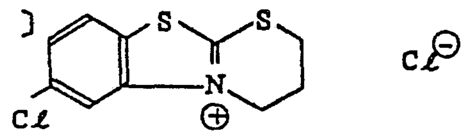

Compounds (A) can be easily synthesized from azole compounds having a methyl mercapto group, as illustrated below.

Synthesis Example 1
(compound A—5)

A mixture of 2-methylthiobenzothiazole (16 g) and 1,3-dibromopropane (25 g) was heated at 160°C for 4 hours. After cooling, the resulting crystals were filtered off and recrystallized from ethanol to produce acicular crystals of the end compound (14 g) having a melting point of 260°C.

Synthesis Example 2
(compound A—7)

Compound [A—7] can be synthesized by the method described in Chemical Abstracts, *72,* 31666 (1970).

The amount of compounds (A) to be used in the present invention varies with the type of binder (e.g. gelatin), the drying conditions used in their preparation, and the composition of silver halide in the specific emulsion, but they are used in an amount of 0.001 to 2 mg per gram of the total binder present in the hydrophilic colloidal layer on the same side of the support as the photosensitive silver halide emulsion layers. An amount of 0.01 to 1 mg is particularly preferred, and the compounds are generally applied as a 0.1 to 1% by weight solution in water or a solvent such as methanol. Compounds (A) may be incorporated in any step prior to the coating and drying steps in the process of preparing the photographic material.

The compound (A) used in the present invention is described in Japanese Patent Publications Nos. 41095/72, 39169/73 and 48172/74 as a material capable of hardening protein, forming a gelatin hardened image or curing a vinyl polymer. But none of these prior art references suggest a method of minimizing the deterioration of a photographic image on a silver halide photographic material that occurs due to the use of the polyalkylene oxide compound which is another feature of the present invention.

In addition, the present invention does not depend on the hardening of a binder such as gelatin for minimizing the above described image deterioration due to the use of the polyalkylene oxide compound. Therefore, the photographic material of the present invention possesses highly stable photographic characteristics that can hardly be accomplished by a gelatin hardener or gelatin hardening method. This finding is quite unexpected in that compound (A) is very effective in minimizing the deterioration of a silver halide photographic image due to the use of the polyalkylene oxide compound, and that this can be achieved without causing any detrimental effects (e.g. increased fog and decreased sensitivity) on the properties of the silver halide photographic material that may be incidental to the use of the hardening of a binder such as gelatin.

Both compound (A) and polyalkylene oxide compound were individually known as components of silver halide emulsions, but it was unexpected that a great advantage would be obtained by combining the two compounds and by using a specific type for each compound.

Various polyalkylene oxide compounds may be combined with compounds (A), and they include polyalkylene glycols, condensation products of aliphatic alcohols, aliphatic amines or aliphatic amides and polyalkylene oxide condensation products of phenols and polyalkylene oxide, condensates of polyalkylene oxide and anhydrohexitol, and addition polymers of polyalkylene oxide and phenol-formalin resins. Any polyalkylene oxide compound having an alkylene oxide chain with an average molecular weight of 250 or more can be used, and those having alkylene oxide chains with an average molecular weight of 500 to 4,000 are preferred. Of course, polyalkylene oxide compounds having alkylene oxide chains with higher molecular weights may be used.

The polyalkylene oxide compounds that can be used in the present invention may be prepared by any of the methods described in prior art references such as Japanese Patent Publications Nos. 13822/68, 8742/72, 11116/72 and 378/65; Japanese Patent Application Nos. 19213/73, 10722/74, 74929/74, 13072/74, 57427/75 and 3219/76; U.S. Patents No. 2,240,472, 1,970,573, 2,400,532, 2,423,549, 2,441,389, 3,017,271, 3,062,647, 2,312,553 and 3,026,202; and British Patent No. 805,826. Typical examples are listed below, with the average molecular weight of each ethylene oxide chain being put within parentheses:

[E—1] polyethylene glycol (400)

[E—2] polyethylene glycol (1,500)

[E—3] a block condensation product of polyethylene oxide and polypropylene oxide (3,000)

[E—4] a condensation product of polyethylene oxide and para-tert-butylphenol (1,350)

[E—5] an additon polymer of polyethylene oxide and nonyl-phenol-formalin resin (500)

[E—6] a condensation product of polyethylene oxide and p-thiocresol (1,500)

[E—7] an addition polymer prepared by adding 1 mol of n-butyl mercaptan to a polymer of polyethylene oxide and polypropylene oxide (1,000)

[E—8] a condensation product of polyethylene oxide and lauryl alcohol (1,250)

[E—9] an addition product of polyethylene oxide and β-ethylene thioethyl isocyanate (600)

[E—10] N-lauryl-N,N-dipolyoxyethylene-N-carboxymethylbetaine (1,000)

[E—11] a condensation product of N-tridecylamide and polyethylene oxide (350)

[E—12] an addition product of polyethylene oxide and octylphenol-formalin resin (800)

[E—13] a condensation product of polyethylene oxide and m-cresol (500)

[E—14] an addition polymer of polyethylene oxide and nonyl-phenol-formalin resin of polyglycidol (500)

[E—15] a sodium sulfonate of an addition product of di-t-octylphenol and polyethylene oxide (650).

The amount of the polyalkylene oxide compounds to be used in the present invention varies with the type of the silver halide emulsion and photographic material used, but preferably, they are used in an amount of 0.1 to 20 g per liter of the coating solution.

Illustrative silver halides that can be incorporated in the silver halide photographic material of the present invention are silver chloride, silver bromide, silver iodide, silver chlorobromide, silver iodobromide, silver chloroiodobromide and mixtures thereof; silver iodobromide is most preferred.

Gelatin is most preferred as a binder or hydrophilic colloid for use in the present invention, but if necessary, gelatin may be used together with, e.g., gelatin derivatives, colloidal albumin, agar, gum arabic, alginic acid, cellulose derivatives, acrylamide, imidated polyacrylamide, casein, and polymers such as vinyl alcohol polymers, polyvinyl alcohol, polyvinyl pyrrolidone or hydrolyzed polyvinyl acetate.

The silver halide photographic emulsion used in the present invention may contain any of the known photographic addenda such as chemical sensitizers, spectral sensitizers, antifoggants, hard contrasting agents, gelatin hardeners, surfactants, film property modifying agents, thickeners and halftone dot modifying agents. Illustrative chemical sensitizers include activated gelatin; noble metal sensitizers such as water soluble gold salts, platinum salts, palladium salts, rhodium salts and iridium salts; sulfur sensitizers; selenium sensitizers; and reduction sensitizers such as polyamine and stannous chloride. These chemical sensitizers may be used either alone or in combination. The spectral sensitizers that can be added are not limited to any particular compounds, and common spectral sensitizers such as cyanine or merocyanin dyes like zero-, mono-, di- and tri-methine dyes may be used either individually or in mixture for optical sensitization. For details of the spectral sensitizers, see various prior art references such as U.S. Patents Nos. 2,688,545, 2,912,329, 3,397,060, 3,615,635, and 3,628,964; British Patents Nos. 1,195,302, 1,242,588 and 1,293,862; DE—A—2,030,326 and 2,121,780; and Japanese Patent Publications Nos. 4936/68 and 14030/69. Suitable sensitizers may be selected depending upon the region of wavelengths for which the emulsion is sensitized, as well as the sensitivity and other factors of the photographic material.

The emulsion for use in the silver halide photographic material of the present invention may contain other additives such as stabilizers or antifoggants (e.g. azaindenes triazoles, tetrazoles, imidazolium salts, tetrazolium salts and polyhydroxy compounds); hardeners (e.g. aldehyde, aziridine, isoxazole, vinyl sulfone, acryloyl, carbodiimide, maleimide, methane, sulfonate ester and triazine compounds); development accelerators (e.g. benzyl alcohol and polyoxyethylene compounds); image stabilizers (e.g. chroman, coumaran, bisphenol and phosphorous acid ester compounds); lubricants (e.g. wax, glyceride of higher aliphatic acids, and higher alcohol esters of higher aliphatic acids). Surfactants may be used as coating aids, agents to improve the permeability to processing solutions, defoamers or agents to control various physical properties of the photographic material, and any of the cationic, anionic, nonionic or amphoteric surfactants may be employed. Suitable antistats are diacetyl cellulose, styrene-perfluoroalkyl lithium maleate copolymers, and alkali salts of the reaction product of styrene-maleic anhydride copolymer and p-aminobenzenesulfonic acid. A latex may be added to provide better film properties, and suitable examples are copolymers of acrylate esters or vinyl esters and other ethylene-containing monomers. Illustrative gelatin plasticizers include glycerin and glycolic compounds, and suitable thickeners are styrene-sodium maleate copolymers and alkylvinylether-maleic acid copolymers.

The silver halide photographic material of the present invention can be formed by coating silver halide photographic emulsions onto supports optionally through subbing layers or intermediate layers, and any known method may be used to apply the silver halide emulsions. Suitable supports include baryta paper, polyethylene-coated paper, synthetic polypropylene paper, glassine paper, cellulose acetate, cellulose nitrate, polyvinylacetal, polypropylene, polyesters such as polyethylene terephthalate, and polystyrene.

The silver halide photographic material of the present invention may be used in either black-and-white or color photography. It may be used for general purposes, for printing, as well as for X-ray and other radiographic applications, and particularly significant advantages are accomplished when the material is used as a high-sensitivity silver bromide photographic material. The silver halide photographic material of the present invention may be subjected to not only ordinary exposure but also to short-time or flash exposure, and it may be processed by the conventional method of photographic processing. Two basic steps are development and fixing, which may be effected simultaneously.

The present invention is hereunder described in greater detail by reference to illustrative examples to which the scope of the invention is by no means limited.

Example 1

A high-sensitivity negative emulsion 1 kg of which contained 40 g of silver iodobromide (containing 3.5 mol% silver iodide) and 45 g of gelatin and which had been subjected to sulfur and gold sensitizations was heated at 40°C and divided into 38 portions. To half of the portions, compounds (A) and polyalkylene oxide compounds were added in the amounts indicated in Table 1. The resulting coating solutions were applied to cellulose triacetate film supports to give a thickness of 4 μm and dried. The melting points of the respective samples were measured in an aqueous solution of 0.2 N sodium hydroxide. Then, the samples were exposed to a white light in a Model KS—1 sensitometer product of Konishiroku Photo Industry Co., Ltd. by the method specified in JIS, and processed by a continuous roller transport type automatic processor that effected development, fixing, washing and drying in sequence. The development period was 30 seconds at 35°C and the developing solution used had the following formulation.

Developing solution

| | |
|---|---|
| Anhydrous sodium sulfite | 70 g |
| Hydroquinone | 10 g |
| Boric anhydride | 1 g |
| Sodium carbonate (monohydrate) | 20 g |
| 1-Phenyl-1,3-pyrazolidone | 0.35 g |
| Sodium hydroxide | 5 g |
| 5-Methyl benzotriazole | 0.05 g |
| Potassium bromide | 5 g |
| Glutaraldehyde bisulfite | 15 g |
| Glacial acetic acid | 8 g |
| Water | to make 1,000 ml |

The so processed samples were subjected to sensitometry to determine their photographic characteristics which are shown in Table 1 wherein the sensititivity is indicated as relative values with that of controls (Samples Nos. 1 and 20) being taken as 100. The image quality was evaluated by visually inspecting the deterioration of developed silver grains at a density of 0.8. The criteria were ○ (good), △ (moderate) and × (very poor). The combination of two of these symbols indicate the state in between those represented by the respective symbols.

TABLE 1

| Sample No. | Compound A Identification No. | Compound A Amount (mg/100g emulsion) | Polyalkyleneoxide compound Identification No. | Polyalkyleneoxide compound Amount (g/lg-gelatin) | m.p. (°C) | Photographic characteristics after standing at 25°C and 60% r.h. Fog | Sensitivity | Image Quality |
|---|---|---|---|---|---|---|---|---|
| 1 | — | — | — | — | 28 | 0.03 | 100 | △ |
| 2 | — | — | E—3 | 0.1 | 〃 | 0.04 | 120 | × |
| 3 | — | — | E—5 | 0.1 | 〃 | 0.04 | 120 | △ × |
| 4 | — | — | E—8 | 0.1 | 〃 | 0.04 | 117 | × |
| 5 | — | — | E—11 | 0.1 | 〃 | 0.04 | 115 | × |
| 6 | — | — | E—15 | 0.1 | 〃 | 0.03 | 115 | × |
| 7 | A—1 | 4.5 | — | — | 〃 | 0.04 | 98 | ○ △ |
| 8 | A—2 | 4.5 | — | — | 〃 | 0.04 | 98 | ○ △ |
| 9 | A—3 | 4.5 | — | — | 〃 | 0.03 | 98 | ○ △ |
| 10 | A—4 | 4.5 | — | — | 〃 | 0.04 | 95 | ○ △ |
| 11 | A—5 | 0.45 | — | — | 〃 | 0.04 | 98 | ○ △ |
| 12 | 〃 | 4.5 | — | — | 〃 | 0.04 | 98 | ○ △ |
| 13 | 〃 | 45 | — | — | 33 | 0.04 | 82 | ○ △ |
| 14 | 〃 | 450 | — | — | 38 | 0.02 | 65 | ○ |
| 15 | A—6 | 4.5 | — | — | 28 | 0.04 | 95 | ○ △ |
| 16 | A—7 | 4.5 | — | — | 〃 | 0.04 | 98 | ○ △ |
| 17 | A—2 | 4.5 | E—3 | 0.1 | 〃 | 0.03 | 120 | ○ △ |
| 18 | A—5 | 0.45 | 〃 | 0.1 | 〃 | 0.03 | 120 | ○ △ |
| 19 | 〃 | 4.5 | 〃 | 0.1 | 〃 | 0.03 | 122 | ○ △ |

As Table 1 shows, the image quality deterioration due to the polyalkylene oxide compounds was effectively eliminated by compounds (A) without increasing the melting points of the samples.

Example 2

An X-ray silver iodobromide emulsion in gelatin that contained 2 mol% of silver iodide was subjected to the second ripening and mixed with saponin as a coating aid. The mixture was then divided into 20 portions. Compounds (A) and polyalkylene oxide compounds were added in the amounts indicated in Table 2. The resulting coating solutions were applied onto polyethylene terephthalate film supports to give a dry thickness of 5 μm and dried. The respective samples were subjected to X-ray sensitometry through a medium-sensitivity fluorescent intensifying screen (product of Toshiba Corp.) and an aluminum step wedge at a tube voltage of 60 kV and a tube current of 200 mA. The samples were then processed as in Example 1 to evaluate their sensitivity and the degree of fog, and the results are shown in Table 2. At the same time, the melting points of the samples were measured as in Example 1 to evaluate the quality of the resulting images. The results are also shown in Table 2.

TABLE 2

| Sample No. | Compound (A) Identification No. | Compound (A) Amount (mg/gelatin) | Polyalkylene Oxide compound Identification No. | Polyalkylene Oxide compound Amount (g/lg-gelatin) | m.p. (°C) | Photographic Characteristics* Fog | Photographic Characteristics* Sensitivity | Image Quality |
|---|---|---|---|---|---|---|---|---|
| 1 | — | — | — | — | 30 | 0.04 | 100 | △ |
| 2 | — | — | E—3 | 0.2 | 〃 | 0.05 | 125 | × |
| 3 | A—5 | 0.01 | 〃 | 〃 | 〃 | 0.04 | 130 | ○ |
| 4 | A—5 | 0.1 | 〃 | 〃 | 〃 | 0.04 | 130 | ○ |
| 5 | A—5 | 0.5 | 〃 | 〃 | 〃 | 0.04 | 125 | ○ |
| 6 | A—5 | 2.0 | 〃 | 〃 | 〃 | 0.04 | 125 | ○ |
| 7 | H—1** | 1 | 〃 | 〃 | 38 | 0.04 | 110 | × |
| 8 | H—1 | 5 | 〃 | 〃 | 50 | 0.04 | 105 | △ × |
| 9 | H—1 | 10 | 〃 | 〃 | 75 | 0.05 | 100 | △ |
| 10 | H—2 | 1 | 〃 | 〃 | 35 | 0.04 | 125 | × |
| 11 | H—2 | 5 | 〃 | 〃 | 46 | 0.04 | 100 | △ × |
| 12 | H—2 | 10 | 〃 | 〃 | 72 | 0.03 | 75 | ○ △ |
| 13 | H—3 | 1 | 〃 | 〃 | 37 | 0.04 | 120 | △ × |
| 14 | H—3 | 5 | 〃 | 〃 | 49 | 0.05 | 120 | △ × |
| 15 | H—3 | 10 | 〃 | 〃 | 75 | 0.08 | 110 | △ |
| 16 | B—1*** | 0.005 | 〃 | 〃 | 30 | 0.04 | 120 | × |
| 17 | B—1 | 0.05 | 〃 | 〃 | 〃 | 0.03 | 95 | × |
| 18 | B—1 | 0.5 | 〃 | 〃 | 〃 | 0.02 | 32 | △ × |
| 19 | B—2 | 0.005 | 〃 | 〃 | 〃 | 0.04 | 125 | × |
| 20 | B—2 | 0.05 | 〃 | 〃 | 〃 | 0.03 | 100 | × |

Footnotes:

*After standing for 3 days at 23°C and 65% r.h.

**Compounds (H—1), (H—2) and (H—3) were hardeners which respectively had the following formulas:

(H-1) $(CHO)_2$ ,

(H-2) 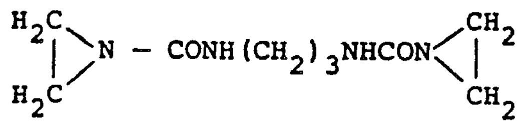 , and

(H-3) 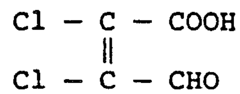

***Compounds (B—1) and (B—2) were development restrainers which respectively had the following formulas:

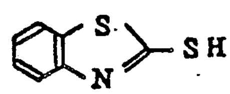 , 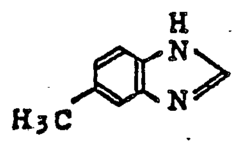

(B-1) (B-2)

As Table 2 shows, the samples according to the present invention had high sensitivities and produced good images with minimum fog. Furthermore, their melting points were not as high as to cause excessive hardening of the emulsion layer.

**Claims**

1. A silver halide photographic material comprising a support and one or more layers thereon wherein at least one layer contains (a) at least one compound having an alkylene oxide chain with an average molecular weight of 250 or more which is a polyalkylene glycol or a condensation product of alkylene oxide and an organic compound having active hydrogen, and wherein at least one layer contains (b) at least one compound of formula (I):

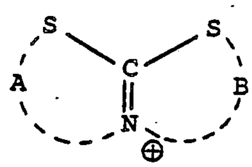 $X^{\ominus}$ I

wherein A and B each independently represents the non-metallic atoms necessary to form a hetero ring; and X is a negative ion in an amount from 0.001 to 2 mg per gram of total binder present in a hydrophilic colloidal layer.

2. A silver halide photographic material according to claim 1, wherein said A or B in formula (I) has the formula:

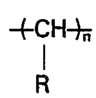

wherein n is 2 or 3; and R is a hydrogen atom or an alkyl group having from 1 to 6 carbon atoms.

3. A silver halide photographic material according to claim 2, wherein n is 2.

4. A silver halide photographic material according to claim 1, wherein said A or B in formula (I) has the formula:

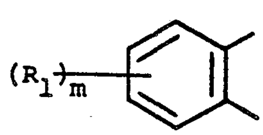

wherein $R_1$ is a hydroxyl group, a halogen atom, an alkyl group which may have a substituent, an aralkyl group which may have a substituent, an acyl group which may have a substituent, a carboxymethyl group which may have a substituent, or a —COOH or —$SO_3H$ group; and m is 0, 1 or 2.

5. A silver halide photographic material according to claim 4, wherein said A or B in formula (I) has the formula:

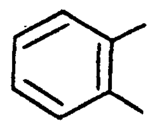

6. A silver halide photographic material according to claim 1, wherein said A in formula (I) has the formula:

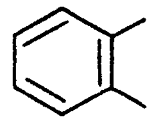

and said B in formula (I) has the formula:

$$-(CH_2)_2-$$

7. A silver halide photographic material according to any one of claims 1 to 6 wherein said organic compound having active hydrogen is an aliphatic alcohol, aliphatic amine, aliphatic amide, phenol, anhydrohexitol, or a phenol-formaldehyde resin.

8. A silver halide photographic material according to any one of claims 1 to 7 wherein the average molecular weight of said alkylene oxide chain is 500 to 4,000.

9. A silver halide photographic material according to any one of the preceding claims wherein said compound of formula (I) is present in an amount from 0.01 to 1 mg per gram of total binder present in a hydrophilic colloidal layer.

10. A silver halide photographic material according to any one of claims 1 to 9 wherein said compound is a polyalkylene glycol.

11. A silver halide photographic material according to any one of claims 1 to 10 which is suitable for X ray use.

**Patentansprüche**

1. Photographisches Silberhalogenid-Aufzeichnungsmaterial mit einem Schichtträger und einer oder mehreren darauf aufgetragenen Schicht(en), von denen mindestens eine

(a) mindestens eine Verbindung mit einer Alkylenoxidkette eines durchschnittlichen Molekulargewichts von 250 oder mehr in Form eines Polyalkylenglykols oder eines Kondensationsprodukts von Alkylenoxid mit einer organischen Verbindung mit aktivem Wasserstoff und mindestens eine Schicht

(b) mindestens eine Verbindung der Formel

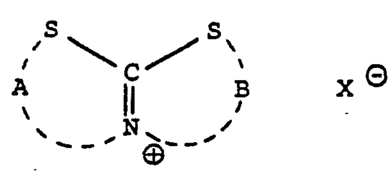 I

worin A und B jeweils für die zur Bildung eines Heterorings erforderlichen nicht-metallischen Atome stehen und X ein negatives Ion darstellt, in einer Menge von 0,001 bis 2 mg pro Gramm des gesamten in einer hydrophilen Kolloidschicht vorhandenen Bindemittels, enthält.

2. Photographisches Silberhalogenid-Aufzeichnungsmaterial nach Anspruch 1, dadurch gekennzeichnet, daß A oder B in Formel (I) der Formel:

$$-(\underset{\displaystyle R}{\underset{|}{CH}})_n-$$

worin n = 2 oder 3 und R für ein Wasserstoffatom oder eine Alkylgruppe mit 1 bis 6 Kohlenstoffatom(en) steht, entspricht.

3. Photographisches Silberhalogenid-Aufzeichnungsmaterial nach Anspruch 2, dadurch gekennzeichnet, daß n = 2.

4. Photographisches Silberhalogenid-Aufzeichnungsmaterial nach Anspruch 1, dadurch gekennzeichnet, daß A oder B in Formel (I) der Formel:

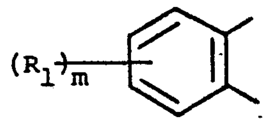

worin $R_1$ eine Hydroxylgruppe, ein Halogenatom, eine gegebenenfalls substituierte Alkylgruppe, eine gegebenenfalls substituierte Aralkylgruppe, eine gegebenenfalls substituierte Acylgruppe, eine gegebenenfalls substituierte Carboxymethylgruppe oder eine —COOH— oder —$SO_3H$-Gruppe darstellt und m = 0, 1 oder 2, entspricht.

5. Photographisches Silberhalogenid-Aufzeichnungsmaterial nach Anspruch 4, dadurch gekennzeichnet, daß A oder B in Formel (I) der Formel:

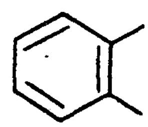

entspricht.

6. Photographisches Silberhalogenid-Aufzeichnungsmaterial nach Anspruch 1, dadurch gekennzeichnet, daß A in Formel (I) der Formel:

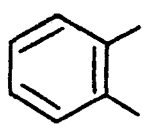

und B in Formel (I) der Formel:

$$-(CH_2)_2-$$

entspricht.

7. Photographisches Silberhalogenid-Aufzeichnungsmaterial nach einem der Ansprüche 1 bis 6, dadurch gekennzeichnet, daß die organische Verbindung mit aktivem Wasserstoff aus einem aliphatischen Alkohol, aliphatischen Amin, aliphatischen Amid, Phenol, Anhydrohexit oder einem Phenol/Formaldehyd-Harz besteht.

8. Photographisches Silberhalogenid-Aufzeichnungsmaterial nach einem der Ansprüche 1 bis 7, dadurch gekennzeichnet, daß das durchschnittliche Molekulargewicht der Alkylenoxidkette 500 bis 4000 beträgt.

9. Photographisches Silberhalogenid-Aufzeichnungsmaterial nach einem der vorhergehenden Ansprüche, dadurch gekennzeichnet, daß die Verbindung der Formel (I) in einer Menge von 0,01 bis 1 mg pro Gramm des gesamten in einer hydrophilen Kolloidschicht enthaltenen Bindemittels vorhanden ist.

10. Photographisches Silberhalogenid-Aufzeichnungsmaterial nach einem der Ansprüche 1 bis 9, dadurch gekennzeichnet, daß die Verbindung aus einem Polyalkylenglykol besteht.

11. Photographisches Silberhalogenid-Aufzeichnungsmaterial nach einem der Ansprüche 1 bis 10 zur Verwendung in der Röntgenphotographie.

**Revendications**

1. Matériau photographique à halogénure d'argent, comprenant un support et une ou plusieurs couches par-dessus, dans lequel au moins une couche contient (a) au moins un composé comportant une chaîne oxyalkylène, d'une masse moléculaire moyenne de 250 ou plus, et qui est un poly(alkylène-glycol) ou un produit de condensation d'un oxyde d'alkylène et d'un composé organique à hydrogène actif, et dans lequel au moins une couche contient (b) au moins un composé de formule (I):

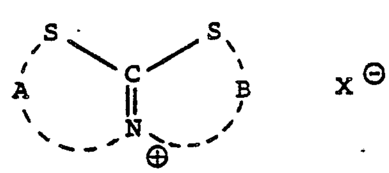

I

dans laquelle A et B représentent chacun, indépendamment, les atomes non métalliques nécessaires pour

former un hétérocycle, et X est un ion négatif, en une quantité comprise entre 0,001 et 2 mg par gramme de la totalité du liant présent dans une couche de colloïde hydrophile.

2. Matériau photographique à halogénure d'argent conforme à la revendication 1, dans lequel A ou B, dans la formule (I), présente la formule:

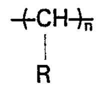

dans laquelle n vaut 2 ou 3, et R est un atome d'hydrogène ou un groupe alkyle comportant de 1 à 6 atomes de carbone.

3. Matériau photographique à halogénure d'argent conforme à la revendication 2, dans lequel n vaut 2.

4. Matériau photographique à halogénure d'argent conforme à la revendication 1, dans lequel A ou B, dans la formule (I), présente la formule:

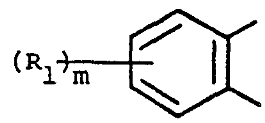

dans laquelle $R_1$ est un groupe hydroxyle, un atome d'halogène, un groupe alkyle éventuellement substitué, un groupe aralkyle éventuellement substitué, un groupe acyle éventuellement substitué, un groupe carboxyméthyle éventuellement substitué, ou un groupe —COOH ou —$SO_3H$, et m vaut 0, 1 ou 2.

5. Matériau photographique à halogénure d'argent conforme à la revendication 4, dans lequel A ou B, dans la formule (I), présente la formule:

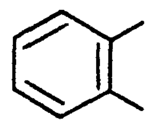

6. Matériau photographique à halogénure d'argent conforme à la revendication 1, dans lequel A, dans la formule (I), présente la formule:

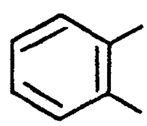

et B dans la formule (I), présente la formule:

$$-(CH_2)_2-$$

7. Matériau photographique à halogénure d'argent conforme à l'une quelconque des revendications 1 à 6, dans lequel ledit composé organique à hydrogène actif est un alcool aliphatique, une amine aliphatique, un amide aliphatique, un phénol, un anhydrohexitol, ou une résine phénol-formaldéhyde.

8. Matériau photographique à halogénure d'argent, conforme à l'une quelconque des revendications 1 à 7, dans lequel la masse moléculaire moyenne de ladite chaîne oxyalkylène est comprise entre 500 et 4000.

9. Matériau photographique à halogénure d'argent, conforme à l'une quelconque des revendications précédentes, dans lequel ledit composé de formule (I) est présent à raison de 0,01 à 1 mg par gramme de la totalité du liant présent dans une couche de colloïde hydrophile.

10. Matériau photographique à halogénure d'argent, conforme à l'une quelconque des revendication 1 à 9, dans lequel ledit composé est un poly(alkylène-glycol).

11. Matériau photographique à halogénure d'argent, conforme à l'une quelconque des revendications 1 à 10, et qui convient pour être utilisé avec des rayons X.